# EUROPEAN PATENT APPLICATION

(11) **EP 2 292 594 A1**
(43) Date of publication of application: **09.03.2011**
(21) Application number: 09750556.4
(22) Date of filing: 19.05.2009
(51) Int. Cl.: C07C 319/20, C07B 57/00, C07C 323/32, C07C 323/63

(54) **METHOD FOR PRODUCING OPTICALLY ACTIVE AMINOALCOHOL DERIVATIVE**

(30) Priority: 19.05.2008 JP 2008130464
(71) Applicant: Kyorin Pharmaceutical Co., Ltd., Tokyo 101-8311 (JP)
(72) Inventor: TSUBUKI Takeshi, Shimotsuga-gun Tochigi 329-0114 (JP); ARAYA Ichiro, Shimotsuga-gun Tochigi 329-0114 (JP); KANDA Takahiro, Shimotsuga-gun Tochigi 329-0114 (JP); KANAZAWA Shintaro, Shimotsuga-gun Tochigi 329-0114 (JP)
(74) Representative: Berryman, Natalia Grace
(86) International application number: PCT/JP2009/059175
(87) International publication number: WO 2009/142194

(57) **Abstract**

Disclosed is a preparation method which makes it possible to produce an aminoalcohol derivative having a high optical purity and to enable the large scale synthesis thereof at a low price.

For instance, a compound represented by the following general formula (8) is recrystallized in the course of the production process thereof

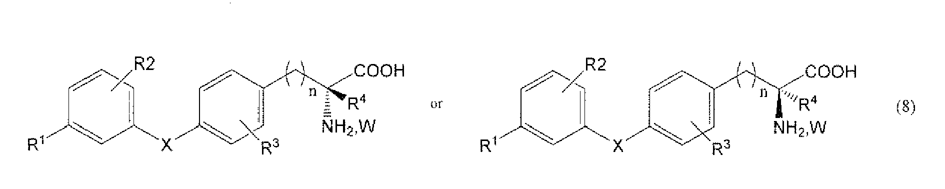

In this case, R¹ represents, for instance, a trihalomethyl group; R² represents, for instance, a hydrogen atom; R³ represents, for instance, a halogen atom; R⁴ represents, for instance, a lower alkyl group; X represents, for instance, a sulfur atom; n represents an integer ranging from 1 to 4; and W represents hydrogen chloride or hydrogen bromine.

## Description

### Technical Field:

The present invention relates to a production method which makes it possible to produce an aminoalcohol derivative having a high optical purity and to also carry out the large scale synthesis of the aminoalcohol derivative at a low cost.

### Background Art

It has been known that aminoalcohol derivatives represented by the following general formula (1) are effective as immune-suppressants (Patent Document 1): 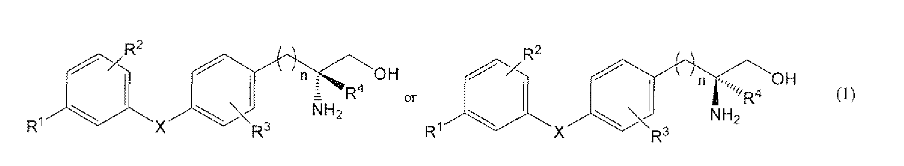 [wherein R¹ represents a halogen atom, a trihalomethyl group, a lower alkyl group having 1 to 4 carbon atoms, an aralkyl group, a lower alkoxy group having 1 to 4 carbon atoms, a phenoxy group which may have a substituent, an aralkyloxy group which may have a substituent, a lower alkylthio group having 1 to 4 carbon atoms, a lower alkylsulfinyl group having 1 to 4 carbon atoms, or a lower alkylsulfonyl group having 1 to 4 carbon atoms;
R² represents a hydrogen atom, a halogen atom, a trihalomethyl group, a lower alkyl group having 1 to 4 carbon atoms, an aralkyl group, a lower alkoxy group having 1 to 4 carbon atoms, or an aralkyloxy group;
R³ represents a hydrogen atom, a halogen atom, a trifluoromethyl group, or a lower alkylthio group having 1 to 4 carbon atoms;
R⁴ represents a hydrogen atom, a lower alkyl group having 1 to 4 carbon atoms, a mono-halogenomethyl group, a lower alkylthiomethyl group having 1 to 4 carbon atoms, a hydroxyethyl group, a hydroxypropyl group, a phenyl group, an aralkyl group, a lower alkenyl group having 2 to 4 carbon atoms, or a lower alkynyl group having 2 to 4 carbon atoms;
X represents an oxygen atom, a sulfur atom, SO, or SO₂; and
n represents an integer ranging from 1 to 4].
In addition, Patent Document 2 discloses that one of the optical isomers thereof is preferred.
On the other hand, as the methods for preparing each of the individual optical isomers of the compound represented by the foregoing general formula (1), there have been known, for instance, synthetic methods comprising the step of optically resolving each of the aminoalcohol derivatives, whose amino group is protected, into the individual isomers thereof using a column for optical resolution (Patent Documents 1 and 2) and a stereo-selective synthetic method which makes use of a chiral- assistant group (Patent Document 2).

Patent Document 1: WO 2004/026817, Pamphlet;
Patent Document 2: WO 2008/018447, Pamphlet.

### Disclosure of the Invention

### Problems That the Invention is to Solve

However, the methods for optically resolving an aminoalcohol derivative, whose amino group is protected, through the use of an optical resolution column never provide each intended optical isomer having a satisfactory optical purity. In addition, the synthetic method using a chiral-assistant group would require a huge cost. Accordingly, it is an object of the present invention to develop a production method which can produce a compound represented by the foregoing general formula (1) at a high optical purity and which permits the large-scale production of such a compound at a low cost.

### Means for the Solution of the Problems

The inventors of this invention have conducted intensive studies to solve the foregoing problems, have found that each of the compounds represented by the general formula (1) having a high optical purity can be prepared by way of a specific amino acid derivative thereof and have thus completed the present invention.
In other words, the present invention thus relates to a method for producing a compound represented by the following general formula (1): 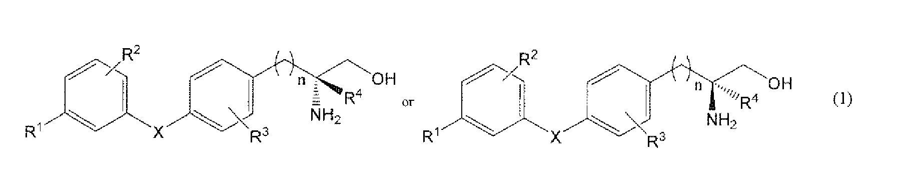 [wherein R¹ represents a halogen atom, a trihalomethyl group, a lower alkyl group having 1 to 4 carbon atoms, an aralkyl group, a lower alkoxy group having 1 to 4 carbon atoms, a phenoxy group which may have a substituent, an aralkyloxy group which may have a substituent, a lower alkylthio group having 1 to 4 carbon atoms, a lower alkylsulfinyl group having 1 to 4 carbon atoms, or a lower alkylsulfonyl group having 1 to 4 carbon atoms;
R² represents a hydrogen atom, a halogen atom, a trihalomethyl group, a lower alkyl group having 1 to 4 carbon atoms, an aralkyl group, a lower alkoxy group having 1 to 4 carbon atoms, or an aralkyloxy group;
R³ represents a hydrogen atom, a halogen atom, a trifluoromethyl group, or a lower alkylthio group having 1 to 4 carbon atoms;
R⁴ represents a hydrogen atom, a lower alkyl group having 1 to 4 carbon atoms, a mono-halogenomethyl group, a lower alkylthiomethyl group having 1 to 4 carbon atoms, a hydroxyethyl group, a hydroxypropyl group, a phenyl group, an aralkyl group, a lower alkenyl group having 2 to 4 carbon atoms, or a lower alkynyl group having 2 to 4 carbon atoms;
X represents an oxygen atom, a sulfur atom, SO, or SO₂; and
n represents an integer ranging from 1 to 4],
the method comprising the steps of:
1. reacting a compound represented by the following general formula (2): 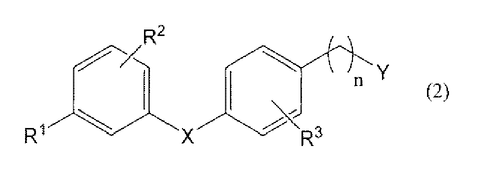 [wherein Y represents a halogen atom, an alkylsulfonyloxy group having 1 to 4 carbon atoms which may have a substituent or an arylsulfonyloxy group which may have a substituent, and R¹, R², R³, X and n are the same as those defined above], with a compound represented by the following general formula (3): 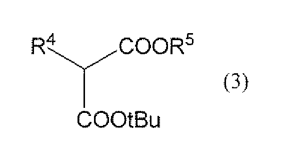 [wherein R⁵ represents a lower alkyl group having 1 to 4 carbon atoms which may have a substituent and R⁴ is the same as that defined above], to thus form a compound represented by the following general formula (4): 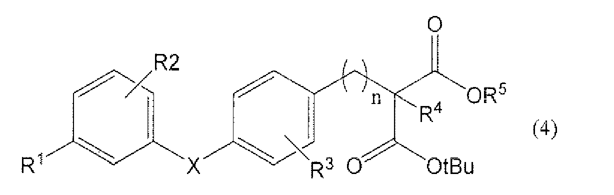 [wherein R¹, R², R³, R⁴ , R⁵, X and n are the same as those defined above], (Step I);
2. replacing the t-butyl group of the compound represented by the general formula (4) with a hydrogen atom to thus form a compound represented by the following general formula (5): 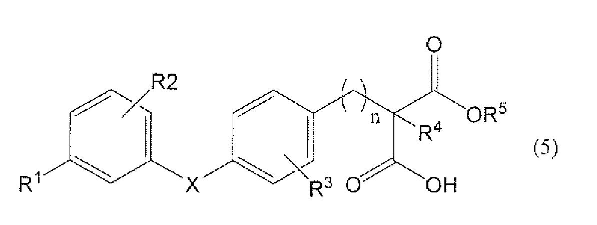 [wherein R¹, R², R³, R⁴, R⁵, X and n are the same as those defined above], (Step II);
3. subjecting the compound represented by the general formula (5) to a rearrangement reaction to thus give a compound represented by the following general formula (6), (Step III): 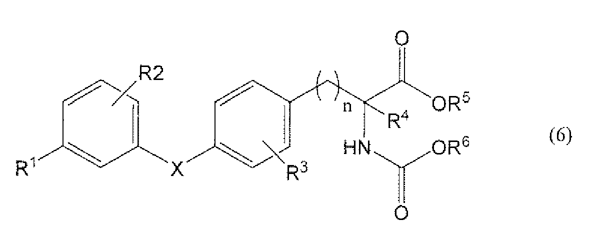 [wherein R⁶ represents a lower alkyl group having 1. to 4 carbon atoms which may have a substituent and R¹, R², R³, R⁴, R⁵, X and n are the same as those defined above];
4. optically resolving the compound represented by the general formula (6) to thus obtain a compound represented by the following general formula (7), (Step IV): 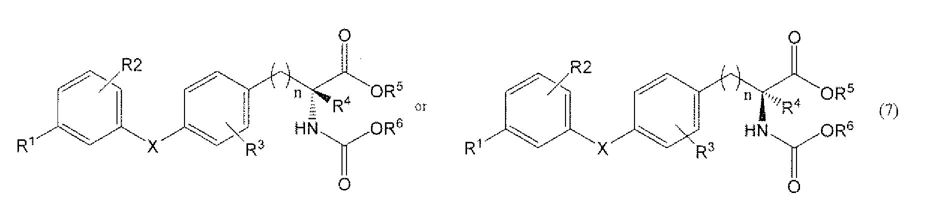 [wherein R¹, R², R³, R⁴, R⁵, R⁶, X and n are the same as those defined above];
5. hydrolyzing the compound represented by the general formula (7) to thus obtain a compound represented by the following general formula (8), (Step V): 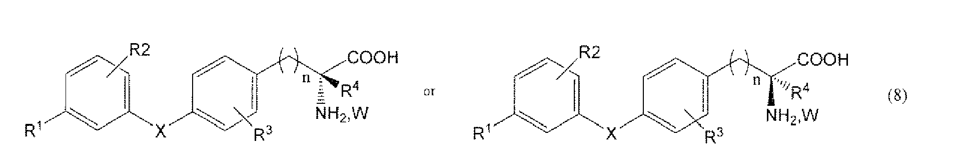 [wherein R², R², R³, R⁴, X and n are the same as those defined above and W represents hydrogen chloride or hydrogen bromide]; and
6. reducing the compound represented by the general formula (8) and then purifying the resulting reduced compound in the form of a salt thereof with fumaric acid or D- or L-tartaric acid to thus give a compound represented by the foregoing general formula (1), (Step VI).

### Effects of the Invention

The method according to the present invention makes it possible to obtain an intended optical isomer having a high optical purity, from the aminoalcohol derivatives represented by the foregoing general formula (1). Moreover, the present invention can also provide a method which can prepare a large quantity of the foregoing desired compound at a low cost.

### Mode for Carrying Out the Invention

The present invention will hereunder be described in more detail.
The term "halogen atom" used in this specification means a fluorine atom, a chlorine atom, a bromine atom or an iodine atom.
The term "trihalomethyl group" used in this specification means a trifluoromethyl group or a trichloromethyl group.
The term "lower alkyl group having 1 to 4 carbon atoms" used in this specification means a linear or branched alkyl group having 1 to 4 carbon atoms and specific examples thereof include methyl, ethyl, propyl, isopropyl, butyl and t-butyl groups.
The term "aralkyl group" used in this specification means, for instance, a benzyl group, a diphenylmethyl group, a phenethyl group, and a phenylpropyl group.

The term "lower alkoxy group having 1 to 4 carbon atoms" used in this specification means a linear or branched alkoxy group having 1 to 4 carbon atoms and specific examples thereof include methoxy, ethoxy, propyloxy, isopropyloxy, butoxy and t-butoxy groups.
The term "aralkyloxy group" used in this specification means, for instance, a benzyloxy group, a diphenylmethyloxy group, a phenethyloxy group, and a phenylpropyloxy group.
The "substituent" of the foregoing "phenoxy group which may have a substituent" or the foregoing "aralkyloxy group which may have a substituent" used in this specification means a fluorine atom, a chlorine atom, a bromine atom, an iodine atom, a trifluoromethyl group, a lower alkyl group having 1 to 4 carbon atoms or a lower alkoxy group having 1 to 4 carbon atoms and the substituent may be on any position of the benzene ring.
The term "lower alkylthio group having 1 to 4 carbon atoms" used in this specification means a linear or branched alkylthio group having 1 to 4 carbon atoms and specific examples thereof include methylthio, ethylthio, propylthio, isopropylthio, butylthio and t-butylthio groups.

The term "lower alkylsulfinyl group having 1 to 4 carbon atoms" used in this specification means a linear or branched alkylsulfinyl group having 1 to 4 carbon atoms and specific examples thereof include methylsulfinyl, ethylsulfinyl, propylsulfinyl, isopropylsulfinyl, butylsulfinyl and t-butylsulfinyl groups.
The term "lower alkylsulfonyl group having 1 to 4 carbon atoms" used in this specification means a linear or branched alkylsulfonyl group having 1 to 4 carbon atoms and specific examples thereof include methylsulfonyl, ethylsulfonyl, propyl sulfonyl, isopropylsulfonyl, butylsulfonyl and t-butylsulfonyl groups.
The term "mono-halogenomethyl group" used in this specification means a methyl group in which one of the hydrogen atoms thereof is replaced with a single halogen atom.

The term "lower alkenyl group having 2 to 4 carbon atoms" used in this specification means, for instance, a vinyl group, an allyl group, a 1-propenyl group, an isopropenyl group, a 1-butenyl group, a 2-butenyl group, a 2-methylallyl group, or a 3-butenyl group.
The term "lower alkynyl group having 2 to 4 carbon atoms" used in this specification means, for instance, an ethynyl group, a 1-propynyl group, a 2-propynyl group, a 1-butynyl group, a 2-butynyl group, or a 3-butynyl group.
The term "alkylsulfonyloxy group having 1 to 4 carbon atoms which may have a substituent" used in this specification means a linear or branched alkylsulfonyloxy group having 1 to 4 carbon atoms, which may be substituted with a halogen atom and specific examples thereof are methanesulfonyloxy and trifluoromethanesulfonyloxy groups.
The term "arylsulfonyloxy group which may have a substituent" used in this specification means an arylsulfonyloxy group which may be substituted with a halogen atom or a lower alkyl group having 1 to 4 carbon atoms and specific examples thereof include benzenesulfonyloxy and toluenesulfonyloxy groups.

As examples of salts of the amino acid derivatives represented by the following general formula (8-b):

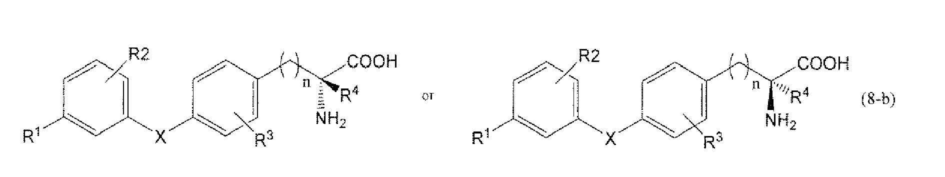

[wherein R¹ represents a halogen atom, a trihalomethyl group, a lower alkyl group having 1 to 4 carbon atoms, an aralkyl group, a lower alkoxy group having 1 to 4 carbon atoms, a phenoxy group which may have a substituent, an aralkyloxy group which may have a substituent, a lower alkylthio group having 1 to 4 carbon atoms, a lower alkylsulfinyl group having 1 to 4 carbon atoms, or a lower alkylsulfonyl group having 1 to 4 carbon atoms;
R² represents a hydrogen atom, a halogen atom, a trihalomethyl group, a lower alkyl group having 1 to 4 carbon atoms, an aralkyl group, a lower alkoxy group having 1 to 4 carbon atoms, or an aralkyloxy group;
R³ represents a hydrogen atom, a halogen atom, a trifluoromethyl group, or a lower alkylthio group having 1 to 4 carbon atoms;
R⁹ represents a hydrogen atom, a lower alkyl group having 1 to 4 carbon atoms, a mono-halogenomethyl group, a lower alkylthiomethyl group having 1 to 4 carbon atoms, a hydroxyethyl group, a hydroxypropyl group, a phenyl group, an aralkyl group, a lower alkenyl group having 2 to 4 carbon atoms, or a lower alkynyl group having 2 to 4 carbon atoms;
X represents an oxygen atom, a sulfur atom, SO, or SO₂; and
n represents an integer ranging from 1 to 4],
there may be listed, for instance, salts with inorganic acids such as hydrochlorides, sulfates, hydrobromides, and phosphates; and salts with organic acids such as acetates, tartarates, maleates, succinates, fumarates, citrates, and p-toluenesulfonates, with hydrochlorides being preferred among others.

Even in case where any satisfactorily high optical purity cannot be attained by the optical resolution technique using an optical resolution column, the method according to the present invention makes it possible to prepare an intended optical isomer represented by the foregoing general formula (1) and having a high optical purity by the combination of such a optical resolution technique with the process for recrystallizing the amino acid derivative [represented by the foregoing general formula (8)].
Moreover, it has been difficult to remove impurities, according to the usual method, from the compounds represented by the general formula (1) obtained after they have been subjected to reducing reactions, but it has been found that such impurities can easily be removed from the compounds when they have been once converted into the fumarates or D- or L-tartarates thereof, and preferably the D- or L-tartarates thereof.

The steps for the preparation of the compounds of Formula (1) according to the present invention will be detailed in the following reaction scheme 1 and each step will be described in more detail below.

### Reaction Scheme 1:

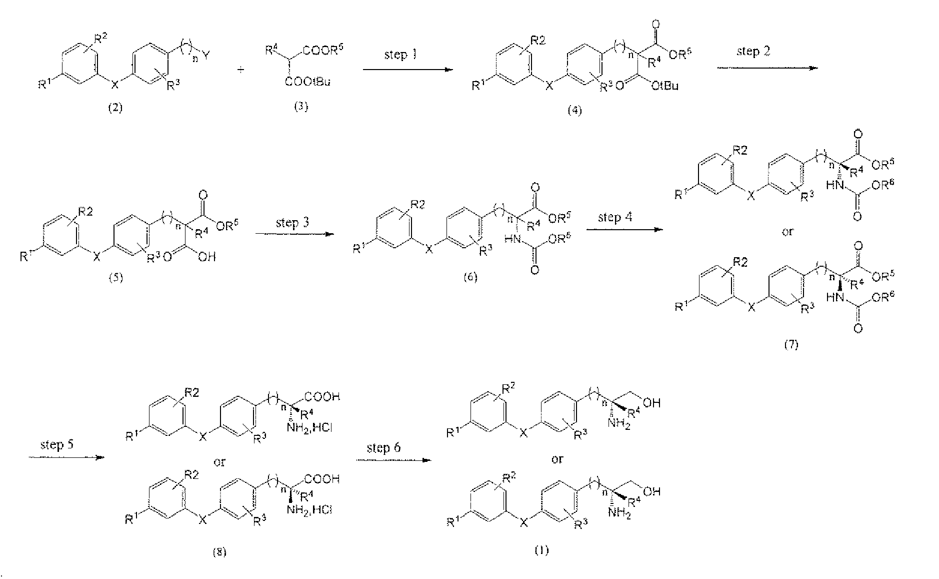

[In the formulas, R¹ , R², R³, R⁴, X and n are the same as those defined above. In this respect, the hydrochlorides of the compounds of Formula (8) may be hydrobromides.]

In the reaction scheme 1, the step 1 is one for condensing a compound represented by the general formula (2) with a compound represented by the general formula (3) to thus form a compound represented by the general formula (4).
The reaction is preferably carried out in the presence of a base. Examples of such bases include inorganic bases such as lithium hydroxide, sodium hydroxide, potassium hydroxide, sodium carbonate, potassium carbonate, cesium carbonate, sodium hydrogen carbonate and potassium hydrogen carbonate; organic bases such as triethylamine, diisopropylethylamine, 4-methylmorpholine, 4-ethylmorpholine, pyridine, 1-methylimidazole, 1,2-dimethylimidazole, 1,5-diazabicyclo[4,3,0]-5-nonene and 1,5-diazabieyelo[5,4,0]-5-undecene; and alkali metal alkoxides such as lithium methoxide, lithium ethoxide, sodium methoxide, sodium ethoxide, sodium t-butoxide, potassium methoxide, potassium ethoxide and potassium t-butoxide; and preferred examples thereof include alkali metal alkoxides and particularly preferred is sodium t-butoxide.

It is suitable in the present invention that the reaction temperature is usually set at a level ranging from -70°C to the boiling temperature of the solvent used, preferably -10°C to the boiling temperature of the solvent used and particularly preferably 20 to 60°C.

The reaction usually requires the use of a solvents and specific examples of such solvents include ethers such as tetrahydrofuran, cyclopentyl methyl ether, dioxane, dimethoxy-ethane and diglyme; aromatic compounds such as benzene, toluene and xylene; hydrocarbons such as hexane, heptane and cyclohexane; nitriles such as acetonitrile and propionitrile; halogenated hydrocarbons such as dichloromethane; alcohols such as methanol, ethanol, t-butyl alcohol, ethylene glycol and diethylene glycol; amides such as formamide, N-methylpyrrolidone and N,N-dimethylformamide; sulfoxides such as dimethylsulfoxide; sulfones such as sulfolane; and mixture thereof, with tetrahydrofuran and N,N-dimethylamide being preferably used herein. A reaction promoter can be added to the foregoing reaction system, and examples thereof include sodium iodide and tetrabutyl-ammonium iodide.

In the foregoing reaction scheme 1, the step 2 is one for replacing the t-butyl group of a compound represented by the foregoing general formula (4) with a hydrogen atom to thus form a compound represented by the foregoing general formula (5) in the form of a monoester.
This reaction is preferably carried out in the presence of an acid. Such acids may be, for instance, silicic acid salts such as Montmorinolite KSF and Montmorinolite K10; organic acids such as p-toluenesulfonic acid, trifluoroacetic acid, methanesulfonic acid and formic acid; and hydrogen chloride solutions such as hydrogen chloride/ethyl acetate and hydrogen chloride/ethanol, with p-toluenesulfonic acid being preferably used herein.
The reaction temperature usually ranges from -70 °C to the boiling temperature of the solvent used, preferably -10°C to the boiling temperature of the solvent used and particularly preferably 60 to 100°C.

The reaction usually requires the use of a solvent and specific examples thereof include ethers such as tetrahydrofuran, cyclopentyl methyl ether, dioxane, dimethoxyethane and diglyme; aromatic compounds such as benzene, toluene and xylene; hydrocarbons such as hexane, heptane and cyclohexane; nitriles such as acetonitrile; halogenated hydrocarbons such as dichloromethane; alcohols such as methanol, ethanol, 2-propanol, t-butyl alcohol, ethylene glycol and diethylene glycol; and mixture thereof, with tetrahydrofuran and toluene being preferably used herein.

It has, in general, been known that when preparing a monoester under acidic conditions, a decarboxylation reaction takes place or proceeds (Non-Patent Document Indian Journal of Chemistry, Section B: Organic Chemistry Including Medicinal Chemistry, 17B(5), 512-513; 1979), a possibility may arise such that a decarboxylated products are formed as impurities, but there was scarcely observed the formation of such decarboxylated products in case of the compounds of the present invention.

In the foregoing reaction scheme 1, the step 3 is one for subjecting a compound represented by the foregoing general formula (5) to a rearrangement reaction to thus form a compound represented by the foregoing general formula (6) as a carbamate derivative thereof.
Examples of such rearrangement reactions usable herein include the Curtius rearrangement reaction, the Schmidt rearrangement reaction, the Lossen rearrangement reaction and the Hofmann rearrangement reaction, with the Curtius rearrangement reaction being preferably used herein.
It would be possible that, with respect to the rearrangement reactions, various conditions may be applied to the respective reaction, but the use of diphenyl phosphoryl azide (DPPA) is preferred when carrying out the Curtius rearrangement reaction.

The carboxylic acid represented by the foregoing general formula (5) can be converted into an acyl azide by the reaction of the same with DPPA in the presence of a solvent, but the acyl azide is thermally rearranged into an isocyanate with ease when it is heated in a solvent. The amount of DPPA to be used is theoretically not less than the equimolar amount relative to the carboxylic acid used, but it preferably ranges from 1 to 3 times the molar amount of the carboxylic acid.
The reaction solvent usable herein may be any inert solvent, but preferably used in the present invention include, for instance, aromatic hydrocarbon solvents such as benzene, toluene and xylene.
The reaction may be carried out, for instance, at a temperature suitably ranging from -10 to 150°C and preferably 60 to 110°C.

The carbamate represented by the foregoing general formula (6) can be prepared by heating this isocyanate in an alcohol, in the presence of a base.
Examples of such bases capable of being used in the reaction include organic bases such as triethylamine, diisopropylethylamine, 4-methylmorpholine, 4-ethylmorpholine, pyridine, 1-methylimidazole, 1,2-dimethylimidazole, 1,5-diazabicyclo-[4,3,0]-5-nonene, and 1,5-diazabicyclo[5,4,0]-5-undecene; and alkali metal alkoxides such as lithium methoxide, lithium ethoxide, sodium methoxide, sodium ethoxide, sodium t-butoxide, potassium methoxide, potassium ethoxide and potassium t-butoxide. The bases used herein are preferably alkali metal alkoxides and particularly preferably sodium ethoxide.

As the alcohols usable in the reaction may be, for instance, methanol, ethanol, propanol, 2-propanol, butanol, and t-butanol and each corresponding product such as methyl carbamate, ethyl carbamate, propyl carbamate, isopropyl carbamate, butyl carbamate or t-butyl carbamate can be obtained depending on the kind of the alcohol used. The amount of the alcohol to be used is theoretically not less than the equimolar amount of each carboxylic acid used, but the alcohol may also serve as a solvent and therefore, it is in general used in a large excess, in such cases.
The reaction temperature usually ranges from -70°C to the boiling temperature of the solvent used, preferably -10°C to the boiling temperature of the solvent used and particularly preferably 0 to 30°C.

In the foregoing reaction scheme 1, the step 4 is one for optically resolving a compound represented by the foregoing general formula (6) to thus give a compound represented by the foregoing general formula (7). The optical resolution thereof can be carried out using a commercially available optical resolution column. It is intended to carry out a large-scale production of each desired compound in the method of the present invention and accordingly, it is preferred in the present invention to carry out the optical resolution according to the simulated moving bed (SMB) technique.

In the foregoing reaction scheme 1, the step 5 is one for the hydrolysis of a compound represented by the general formula (7) to thus obtain an amino acid derivative represented by the foregoing general formula (8). This step can in general be carried out under basic conditions, but in case wherein the substituent R⁶ represents a t-butyl group, the hydrolysis is preferably carried out according to a two-stage reaction, in other words, the compound represented by the general formula (7) is first hydrolyzed with a base and it is subsequently hydrolyzed with an acid.
Examples of such bases used in the hydrolysis reaction include inorganic bases such as lithium hydroxide, sodium hydroxide, potassium hydroxide, barium hydroxide, sodium carbonate, potassium carbonate, cesium carbonate, sodium hydrogen carbonate, and potassium hydrogen carbonate, with the use of sodium hydroxide as such a base being preferred.
Examples of such acids used in the hydrolysis reaction are organic acids such as trifluoromethanesulfonic acid, trifluoroacetic acid, methanesulfonic acid, and formic acid; and inorganic acids such as hydrochloric acid and sulfuric acid, with hydrochloric acid being preferably used, among others.

The reaction temperature usually ranges from -70 °C to the boiling temperature of the solvent used, preferably -10°C to the boiling temperature of the solvent used and particularly preferably 80 to 120°C.
The hydrolysis reaction usually requires the use of a solvent and specific examples thereof include ethers such as tetrahydrofuran, cyclopentyl methyl ether, dioxane, dimethoxy-ethane and diglyme; aromatic compounds such as benzene, toluene and xylene; nitriles such as acetonitrile and propionitrile; halogenated hydrocarbons such as dichloromethane; alcohols such as methanol, ethanol, t-butyl alcohols, ethylene glycol and diethylene glycol; amides such as formamide, N-methylpyrrolidone and N,N-dimethylformamide; sulfoxides such as dimethyl-sulfoxide; sulfones such as sulfolane; and mixture thereof, with dimethyl-sulfoxide or sulfolane being preferably used in the reaction.

In this step, when the amino acid derivative represented by the general formula (8) is purified through the recrystallization thereof, the optical purity of the resulting product can be improved. More specifically, the recrystallization of the compound makes it possible to prepare the same having a desired optical purity which has never been able to be attained by the optical resolution through the use of the optical resolution column.
The solvent used in the recrystallization is not restricted to any specific one insofar as it never undergoes a reaction with the compound represented by the general formula (8) and it is appropriately selected while taking into consideration the intended optical purity and rate of recovery of the compound after the purification. Specific examples of such solvents used for the recrystallization include aliphatic hydrocarbons such as n-pentane, n-hexane, cyclohexane, and n-heptane; aromatic hydrocarbons such as benzene, toluene, ethylbenzene, xylene and mesitylene; halogenated hydrocarbons such as methylene chloride, chloroform, and 1,2-dichloroethane; ethers such as diethyl ether, tetrahydrofuran, t-butyl methyl ether, and 1,4-dioxane; ketones such as acetone, methyl ethyl ketone, and methyl isobutyl ketone; esters such as ethyl acetate and n-butyl acetate; nitriles such as acetonitrile and propionitrile; alcohols such as methanol, ethanol, n-propanol, 2-propanol and n-butanol; and water. These solvents can be used alone or in any combination. Among them, preferably used herein are combinations of, for instance, alcohols such as methanol, ethanol, n-propanol, 2-propanol or n-butanol and water. In particular, the use of a 2-propanol/water mixed solvent is more preferred in the present invention.

In the foregoing reaction scheme 1, the step 6 is one for reducing a compound represented by the general formula (8) to thus give an aminoalcohol derivative represented by the general formula (1).
The reducing method is not restricted to any particular one inasmuch as it is the usual reducing method in which a carboxylic acid is converted into the corresponding alcohol.
Examples of reducing agents used in this step include aluminum hydride type ones such as lithium aluminum hydride, bis(2-methoxy-ethoxy) sodium aluminum hydride, trimethoxy lithium aluminum hydride, aluminum hydride, triethyl boron lithium hydride and diisobutyl aluminum hydride; boron hydride type ones such as borane tetrahydrofuran complex, borane dimethylsulfide complex and diborane; and the combinations of sodium boron hydride with Lewis acids and inorganic salts such as zinc chloride, boron trifluoride, trimethylsilyl halides, lithium chloride, aluminum chloride and cobalt chloride. Preferred examples of the reducing methods are those which make use of sodium boron hydride in the presence of boron trifluoride or borane tetrahydrofuran complex, among others.

The reaction temperature usually ranges from -70 °C to the boiling temperature of the solvent used, preferably -10°C to the boiling temperature of the solvent used and particularly preferably 30 to 50°C.
When using, as a reducing agent, sodium boron hydride in the presence of boron trifluoride, the amount of the sodium boron hydride used in the presence of boron trifluoride in general ranges from 1.0 to 20 times the molar amount of the compound represented by the foregoing general formula (8) and preferably 3.0 to 5.0 times the molar amount thereof.
The reducing reaction usually requires the use of a solvent and specific examples thereof include ethers such as tetrahydrofuran, cyclopentyl methyl ether, dioxane, dimethoxyethane and diglyme; aromatic compounds such as benzene, toluene and xylene; hydrocarbons such as hexane, heptane and cyclohexane; and mixture thereof. Preferred examples thereof include ethers and more preferably dimethoxyethane.

The product obtained after the reducing reaction can easily be purified through the crystallization thereof by the addition of fumaric acid or D- or L-tartaric acid, preferably D- or L-tartaric acid to the product. In this case, the tartaric acid used may be either D-isomer or L-isomer thereof. The solvent used for the crystallization is not limited to any particular one inasmuch as it never undergoes any reaction with the compound represented by the general formula (1) and it is appropriately selected while taking into consideration the intended chemical purity and rate of recovery of the compound after the purification. Specific examples of such solvents used for the recrystallization include aliphatic hydrocarbons such as n-pentane, n-hexane, cyclohexane, and n-heptane; aromatic hydrocarbons such as benzene, toluene, ethylbenzene, xylene and mesitylene; halogenated hydrocarbons such as methylene chloride, chloroform, and 1,2-dichloroethane; ethers such as diethyl ether, tetrahydrofuran, t-butyl methyl ether, and 1,4-dioxane; ketones such as acetone, methyl ethyl ketone, and methyl isobutyl ketone; esters such as ethyl acetate and n-butyl acetate; nitriles such as acetonitrile and propionitrile; alcohols such as methanol, ethanol, n-propanol, 2-propanol and n-butanol; and water. These solvents can be used alone or in any combination. Among them, preferably used herein are combinations of, for instance, alcohols such as methanol, ethanol, n-propanol, 2-propanol or n-butanol and water. In particular, the use of ethanol or a mixed solvent of ethanol and water is more preferred in the present invention.

### EXAMPLES

The preparation method according to the present invention will now be described below with reference to the following Examples, but the present invention is not restricted to these specific Examples at all.

### (Step 1)

To a 35 L volume stainless steel container (with a lid), there were added 714 g (3.53 mol) of t-butyl ethyl 2-methylmalonate and 4.50 L of N,N-dimethylformamide (DMF), to dissolve the former in the latter with stirring and the atmosphere of the solution was replaced with nitrogen gas. The solution was cooled at an external temperature of 7°C, 328 g (3.41 mol) of sodium t-butoxide was added to the solution in small portions (while setting the internal temperature at a level ranging from 8 to 13°C), and then the mixture was subsequently heated at an external temperature of 32°C. After confirming the complete dissolution (the dissolution thereof was completed at an internal temperature of 30°C), the resulting solution was stirred at an internal temperature ranging from 30 to 31°C for 15 minutes. Then, there were added, to the solution, 353 g (2.35 mol) of sodium iodide and 1.00 kg (2.35 mol) of 3-[2-chloro-4-(3-trifluoromethylphenylthio)phenyl]propylmethanesulfonate, followed by the washing thereof with 500 mL of DMF, the replacement of the atmosphere of the solution with nitrogen gas, the heating of the same at an external temperature ranging from 52 to 57°C and the stirring of the solution at an internal temperature ranging from 50 to 56°C for 3.5 hours (while replacing the atmosphere with nitrogen at intervals of 30 minutes).
The solution was cooled at an external temperature of 25°C (at an internal temperature of 25°C), there were added, to the solution, 10.0 L of ethyl acetate and 15.0 L of water, followed by the stirring of the mixture, the adjustment of the pH of the mixture to 7.0 with the use of a diluted hydrochloric acid solution (the volume ratio: hydrochloric acid/water =15.0 mL: 45.0 mL) (the amount of the diluted hydrochloric acid used: 27.0 mL) and the subsequent isolation of the organic phase. The organic phase was washed with a common salt solution (the ratio of common salt to water of 1.50 kg. 5.00L) and then the organic phase was concentrated under reduced pressure (while setting the external temperature at 60°C). The resulting concentrate was added to and dissolved in 3.00 L of toluene and then the resulting solution was concentrated under reduced pressure (while setting the external temperature at 60°C) to thus obtain 1.52 kg of t-butylethyl 2-{3-[2-chloro-4-(3-trifluoromethylphenylthio)phenyl]propyl}-2-methyl-propane dioate as a faintly yellow-colored oily product. The resulting compound was used in the subsequent step without subjecting the same to any particular purification.

### (Step 2)

A Dean-Stark tube was attached to a 10 L volume four-necked flask, there were added, to the flask, 1.52 kg (corresponding to 2.35 mol) of t-butylethyl 2-{3-[2-chloro-4-(3-trifluoromethylphenylthio)phenyl]propyl-2-methylpropane dioate, 5.00 L of toluene and 89.5 g (471 m mol) of p-toluenesulfonic acid monohydrate, and then the mixture was stirred for 2 hours while purging the interior of the flask with nitrogen at an internal temperature ranging from 100 to 112°C, and the solvent was distilled off (the amount of the solvent thus distilled off: 1.21 L). At this point, toluene was freshly supplemented to the resulting residue in an amount identical to that of the solvent distilled off above (the amount of toluene supplemented: 1.21 L)
The resulting mixture was cooled at an external temperature of 25°C (at an internal temperature of 23°C), the reaction solution was transferred to a 48 L volume stainless steel container, 5.00 L of toluene and a common salt solution (100 g of common salt: 10,0 L of water) were added to the reaction solution for the extraction to thus isolate the organic phase. After washing the organic phase with a common salt solution (1.5 kg of common salt: 5.00 L of water), the organic phase was concentrated under reduced pressure (while setting the external temperature at 60°C). To the resulting concentrate, there was added 3.00 L of toluene to dissolve the same, and then the resulting solution was concentrated under reduced pressure (while setting the external temperature at 60°C) to thus obtain 1.22 kg of ethyl hydrogen 2-{3-[2-chloro-4-(3-trifluoromethylphenylthio)phenyl]propyl}-2-methyl- propane dioate as a pale yellow-colored oily product. The resulting compound was used in the next step without subjecting the same to any particular purification.

### (Step 3)

To a 18 L volume stainless steel container, there were added 1.22 kg (corresponding to 2.35 mol) of ethyl hydrogen 2-{3-[2-chloro-4-(3-trifluoromethylphenylthio)phenyl]propyl}-2-methyl-propane dioate and 2.80 L of toluene to thus dissolve the same with stirring, 357 g (3.53 mol) of triethylamine was added to the resulting solution (exothermic: the internal temperature was increased from 25°C to 38°C) and then the mixture was cooled at an external temperature of 25°C. To the cooled mixture, there was slowly dropwise added 842 g (3.06 mol) of diphenyl phosphoryl azide (DPPA) (the internal temperature ranged from 27 to 31°C), the container was washed with 200 mL of toluene, heated at an external temperature of 32°C and then stirred at an internal temperature ranging from 30 to 32°C for one hour to thus give an acid azide solution.
Separately, to a 35 L volume stainless steel container (with a lid), there was added 6.00 L of toluene, and it was heated and stirred at an external temperature of 77°C. The foregoing acid azide solution was slowly dropwise added to the container at an internal temperature ranging from 75 to 78°C (the time of the dropwise addition: 48 minutes), the container was washed with 1.00 L of toluene and then stirred at an internal temperature ranging from 75 to 78°C for 1.5 hours.

The content of the container was cooled at an external temperature of 5°C, there was dropwise added, to the content, a mixed liquid comprising 1.44 kg (4.24 M) of a 20% sodium ethoxide/ethanol solution and 2.00 L of ethanol at an internal temperature ranging from 7 to 14°C, and the content of the container was washed with 500 mL of ethanol and stirred at an internal temperature ranging from 8 to 9°C for 45 minutes.
The reaction liquid was transferred to a 60 L volume stainless steel container, 15.0 L of water was added thereto, and a diluted hydrochloric acid solution (comprising 392 mL (4.71 mol) of hydrochloric acid and 1.00 L of water) was added to the mixture with stirring to thus adjust the pH value thereof to 6.0 (as determined using a pH meter) (the remaining amount of the diluted hydrochloric acid solution: 190 mL). To the mixture, there was added 10.0 L of ethyl acetate for extraction and the resulting organic phase was separated. After washing the organic phase with an aqueous common salt solution (comprising 1.50 kg of common salt and 5.00 L of water), the organic phase was concentrated under reduced pressure (while setting the external temperature at 60°C). To the resulting concentrate, there was added 3.00 L of 2-propanol (IPA) for the dissolution of the concentrate and then the resulting solution was concentrated under reduced pressure (while setting the external temperature at 60°C) to thus obtain a pale brown-colored oily product.
To the resulting pale brown-colored oily product, there were added 3.00 L of hexane and 2.00 L of IPA for the dissolution of the oily product, 100 g of activated carbon was added to the resulting solution and then the mixture was stirred at an internal temperature ranging from 21 to 22°C for 10 minutes. The activated carbon was filtered off, followed by the washing of the same with a mixed liquid containing 3.00 L of hexane and 2.00 L of IPA and the subsequent concentration of the filtrate under reduced pressure (while setting the external temperature at 60°C) to thus obtain 1.26 kg of ethyl 5-[2-chloro-4-(3-trifluoromethylphenylthio)phenyl]-2-ethoxycarbonylamino-2-methylpentanoate as a pale yellow-colored oily product. The resulting compound (1.26 kg) was then optically resolved without subjecting the same to any particular purification.

### (Step 4)

The ethyl 5-[2-chloro-4-(3-trifluoromethylphenylthio)phenyl]-2-ethoxycarbonylamino-2-methyl pentanoate (1.25 kg) was optically resolved according to the simulated moving bed (SMB) technique under the optical resolution conditions specified below to thus give 622 g of ethyl (+)-5-[2-chloro-4-(3-trifluoromethyl phenylthio)phenyl]-2-ethoxycarbonylamino-2-methyl pentanoate.

### Conditions for the SMB Optical Resolution:

Column used: CHIRAL CEL^{®} OD;
Size : 3 cm I.D. × 10 cm L×8;
Moving Phase: n-hexane/IPA= 80/20 (v/v);
Temperature: 40°C;
Zone Construction: 2-3-2-1
Flow Rate of Feed: 4,8 mL/min;
Flow Rate of Elute: 45.8 mL/min;
Flow Rate of Raffinate: 13.0 mL/min;
Flow Rate of Extract: 37.6 mL/min;
Circulation Flow Rate: 100.0 mL/min;
Period of Time: 1.1 minute.

### (Step 5)

To a 10 L volume four-necked flask, there were added 622 g of (+)-ethyl 5-[2-chloro-4-(3-trifluoromethylphenylthio)-phenyl-2-ethoxycarbonylamino-2methyl pentanoate [using 1.00 kg (2.35 mol) of the intermediate (7) in the 5^{th} step] and 3.00 L of dimethyl sulfoxide (DMSO), with stirring, for the dissolution of the former, followed by the addition of 1.50 L of water and 282 g (7.06 mol) of sodium hydroxide and the subsequent stirring of the resulting mixture at an internal temperature ranging from 100 to 116°C for 4 hours. Thereafter, the mixture was cooled at an external temperature of 50°C for the separation of precipitates, 3.00 L of water was added thereto at an internal temperature of 80°C to dissolve the solids thus separated to thus give a solution to be used in the subsequent hydrolysis (the internal temperature: 51°C).
Separately, there were added, to a 35 L volume enameled container, 3.00 L of water and 3.00 L of IPA, the mixture was heated at an external temperature of 50°C with stirring and then 765 mL (9.18 mol) of hydrochloric acid was added to the mixture at an internal temperature of 50°C. To the resulting mixture, there was added the foregoing solution for hydrolysis, the container was washed with 3.00 L of water and then the washing liquid was combined with the foregoing mixture (in this respect, crystal-separation was initiated in the middle of the dropwise addition of the washing liquid and therefore, the dropwise addition was interrupted for 10 minutes, before the addition of the remaining washing liquid). The combined liquid was stirred at an internal temperature ranging from 48 to 50°C for 10 minutes, then it was confirmed that the pH value thereof (determined using a pH meter) was 1.3, the liquid was slowly cooled, stirred at an internal temperature of not higher than 15°C for 30 minutes (the internal temperature was changed from 15°C to 3°C), and the precipitated crystals were filtered off. The crystals were washed with a cooled (internal temperature: 8°C) mixed liquid containing 600 mL of IPA and a diluted hydrochloric acid solution (1.2 mL of hydrochloric acid: 1.20 L of water), and then the liquid was removed from the same to thus give 545 g of crude and wet crystals. The resulting crude and wet crystalline product was dried at 50°C for one hour using an air-blower and further dried at 60°C overnight likewise using an air-blower to thus obtain 431 g of white flaky crude crystals.

To a 10 L volume four-necked flask, there were added 431 g (949 m mol) of the crude crystals, 2.59 L of IPA, 3.45 L of water and 114 g (2.85 mol) of sodium hydroxide and the mixture was heated with stirring at an external temperature of 50°C to thus prepare a solution (at the internal temperature of 41°C, the complete dissolution of the solutes was confirmed). Then the pH value of the resulting solution was adjusted to a level of 1.3 to 1.4 (as determined using a pH meter) (after stirring for 10 minutes, the pH value thereof changed from 1.4 to 1.3) at an internal temperature ranging from 50 to 54°C by the addition of a diluted hydrochloric acid solution (309 mL (3.71 mol) of hydrochloric acid: 309 mL of water) (the remaining amount of the diluted hydrochloric acid solution was found to be 96 mL). The solution was cooled at an external temperature of 35°C to precipitate crystals (the precipitation was initiated at an internal temperature of 39°C), the solution was stirred at a temperature around the crystallization temperature (the internal temperature was changed from 39 to 37°C) for 10 minutes, heated at an external temperature of 53°C (the heating was continued till the internal temperature reached 48°C; about half of the crystals were dissolved), thereafter gradually cooled and stirred at an internal temperature of not higher than 15°C for 30 minutes (the internal temperature was changed from 15 to 7°C). The crystals thus formed were filtered off, washed with a cooled (the internal temperature: 6°C) mixed liquid comprising 431 mL of IPA and a diluted hydrochloric acid solution (0.86 mL of hydrochloric acid: 863 mL of water), and the liquid was removed to thus obtain 535 g of a recrystallized wet product. The resulting recrystallized wet product (535 g) was dried at 50°C for 2 hours using an air-blower and it was further dried at 60°C overnight likewise using an air-blower to thus obtain 386 g of (-)-2-amino-5-[2-chloro-4-(3-trifluoromethylphenylthio)phenyl]-2-methylpentane hydrochloride as a white flaky product.

### (Step 6)

To a 5 L volume four-necked flask, there were added 380 g (836 m mol) of hydrochloride of (-)-2-amino-5-[2-chloro-4-(3-trifluoromethylphenylthio)-phenyl]-2-methylpentane and 1.52 L of 1,2-dimethoxyethane (DME), followed by the addition of 63.3 g (1.67 mol) of sodium boron hydride with heating and stirring at an external temperature of 35°C (exothermic; the internal temperature was changed from 33°C to 52°C). After cooling the resulting mixture to an internal temperature of 35°C, a mixed liquid comprising 210 mL (1.67 mol) of boron trifluoride-diethyl ether complex and 380 mL of DME was slowly dropwise added thereto at an internal temperature ranging from 35 to 41°C (over 67 minutes), the mixture was stirred at an internal temperature ranging from 39 to 40°C for 2 hours, then cooled to an external temperature of 20°C (the internal temperature was found to be 25°C) and the resulting solution was used as a reaction solution.

Separately, to a 30 L volume enameled container, there was added 4.56 L of water, the content of the container was cooled at an external temperature of 5°C (internal temperature: 4°C) with stirring, the reaction solution prepared above was dropwise added to the container, the container was washed with 760 mL of water and the washing liquid was combined with the resulting mixture. A diluted hydrochloric acid solution (348 mL (4.18 mol) of hydrochloric acid: 348 mL of water) was slowly dropwise added to the mixture, thereafter the resulting mixture was heated at an external temperature of 45°C, then stirred at an internal temperature ranging from 35 to 45°C for one hour, cooled at an external temperature of 23°C (internal temperature: 25°C) and then allowed to stand overnight. The mixture was then cooled and stirred at an external temperature of 5 °C (internal temperature: 5°C), the pH value of the mixture was controlled to 8.5 (determined using a pH meter) using an aqueous sodium hydroxide solution (240 g (6.00 mol) of sodium hydroxide: 3.00 L of water) (the pH value observed after stirring the same for 10 minutes: 8.5) (the amount of the remaining aqueous sodium hydroxide solution: 220 mL). To the mixture, there was added 4.56 L of ethyl acetate for extraction and the resulting organic phase was isolated. The organic phase was washed with an aqueous sodium carbonate solution (190 g of sodium carbonate: 3.80 L of water) and an aqueous common salt solution (190 g of common salt: 3.80 L of water) in this order and then concentrated under reduced pressure (the external temperature was set at 45°C). To the resulting concentrate, there was added 1.14 L of ethanol for the dissolution thereof and the resulting solution was concentrated under reduced pressure (while setting the external temperature at 60°C) to thus obtain 354 g of crude (+)-2-amino-5-[2-chloro-4-(3-trifluoromethylphenylthio) phenyl]-2-methyl-1-pentanol as a pale purplish red-colored oily product.

Ethanol (1.14 L) was added to 354 g of the resulting crude (+)-2-amino-5-[2-chloro-4-(3-trifluoromethylphenylthio)phenyl]-2-methyl-1-pentanol to dissolve the latter in the former and there were then added, to the resulting solution, a solution of 138 g (920 m mol) of D-tartaric acid in 1.14 L of water and 1.14 L of water, in this order, with heating and stirring at an external temperature of 54°C(at an internal temperature ranging from 50 to 51°C). The seed crystals of the intended compound were added to the resulting mixture, it was confirmed that the crystals were separated from the same (the crystals were separated from the mixture at an internal temperature of 49°C), the mixture was stirred at an internal temperature of 49°C for 10 minutes, then gradually cooled and stirred at an internal temperature of not higher than 15°C for 30 minutes (the internal temperature was changed from 15°Cto 9°C). The crystals thus separated were filtered off, followed by the washing of the same with an ethanol-water mixed liquid (114 mL of ethanol: 1.03 L of water) and the subsequent removal of the liquid to thus give 605 g of crude and wet crystals of the desired D-tartarate. The resulting crude and wet crystals of the D-tartarate (605 g) was dried at 50°C overnight using an air-blower and further dried at 60°C for one hour and 18 minutes likewise using an air-blower to thus obtain 483 g of crude crystals of the D-tartarate.

To a 10 L volume four-necked flask, there were added 483 g of the crude crystals of the D-tartarate, 4.83 L of IPA and 242 mL of water, and the crude crystals were thus dissolved by heating and stirring the mixture at an external temperature of 75 °C (in this respect, it was confirmed that the crystals were completely dissolved at an internal temperature of 67°C). The resulting solution was cooled at an external temperature of 40°C, it was then confirmed that the crystals were separated from the solution (the crystals were formed at an internal temperature of 42°C), followed by the stirring of the solution containing the crystals at an internal temperature ranging from 41 to 42°C for 10 minutes and the subsequent heating and stirring of the same at an external temperature of 57°C (in this regard, the heating was continued till the internal temperature reached 53°C). Thereafter, the mixture was gradually cooled and it was then stirred at an internal temperature of not higher than 15°C for 30 minutes (the internal temperature was changed from 15°C to 7°C). The crystals thus separated were filtered off, washed with 1.45 L of IPA, the liquid was then removed to thus give 584 g of recrystallized wet crystals of the D-tartarate. The resulting recrystallized wet crystals of the D-tartarate (584 g) were dried at 45°C for 2 hours using an air-blower and further dried at 60°C overnight likewise using an air-blower to thus give 434 g (735 m mol) of the intended D-tartarate.

To a 35 L volume stainless steel container, there were added 434 g (735 m mol) of the D-tartarate and 5.21 L of ethyl acetate, then an aqueous solution of sodium carbonate (312 g of sodium carbonate. 6.08 L of water) was added to the mixture while cooling and stirring the same at an external temperature of 7°C and the resulting mixture was stirred at an internal temperature of not higher than 15°C for 30 minutes (the internal temperature was changed from 15°C to 8°C), followed by the extraction of the product. The resulting organic phase was isolated, washed with an aqueous common salt solution (217 g of common salt: 4.34 L of water) and then concentrated under reduced pressure (while setting the external temperature at 45°C). The concentrate thus obtained was dissolved in 1.30 L of acetonitrile and the resulting solution was concentrated under reduced pressure (while setting the external temperature at 60°C) to thus obtain 303 g of a colorless oily product.
The resulting oily product (303 g) was dissolved in 1-30 L of acetonitrile, 1.30 L of water out of 3.91 1, of water was first added to the solution while cooling and stirring the solution at an external temperature of 5°C, it was confirmed that the crystals were separated from the mixture (the crystallization was initiated at an internal temperature of 8°C), followed by the stirring of the mixture for 10 minutes, the dropwise addition of 2.61 L of the remaining water, the heating of the same at an external temperature of 25 °C , and the stirring thereof at an internal temperature of 25°C for 30 minutes. The mixture was gradually cooled, followed by the stirring of the same at an internal temperature of not higher than 15°C for 30 minutes (the internal temperature was changed from 15°C to 8°C), the removal of the isolated crystals through filtration, the washing of the crystals with an aqueous acetonitrile solution (87 mL of acetonitrile: 1.22 L of water) and the removal of the liquid to thus give 487 g of a wet product of the 11^{th} step-intermediate (13). The resulting wet product (487 g) was dried at 35°C overnight using an air-blower and then dried at 50°C for 3 hours and 58 minutes likewise using an air-blower to thus give 292 g (723 mmol; yield: 86%) of (+)-2-amino-5-[2-chloro-4-(3-trifluoromethylphenylthio) phenyl]-2-methyl-1-pentanol as white powdery crystals.

To a 10 L volume flask used for an evaporator, there were added 240 g (594 m mol) of the (+)-2-amino-5-[2-chloro-4-(3-trifluoromethylphenylthio)phenyl]-2-methyl-1-pentanol prepared above and 1.20 L of ethanol for the dissolution of the compound, followed by the addition, to the resulting solution, a diluted hydrochloric acid solution (49.5 mL (594 m mol) of hydrochloric acid 49.5 mL of purified water) and the subsequent concentration of the mixture under reduced pressure (while setting the external temperature at 45 °C). The resulting concentrate was dissolved in 720 mL of ethanol, the solution was then concentrated under reduced pressure (while setting the external temperature at 60°C), the resulting concentrate was again dissolved in 720 mL of ethanol, the solution was again concentrated under reduced pressure (setting the external temperature at 60°C), thereafter the resulting concentrate was further dissolved in 720 mL of ethanol and the solution was then concentrated under reduced pressure (while setting the external temperature at 60°C), and then the resultant concentrate was dissolved in 720mL of ethyl acetate and was concentrated under reduced pressure (setting the external temperature at 60°C). To the resulting residue, there were added 240 mL of ethyl acetate and 480 mL of isopropyl ether (IPE), followed by the dissolution of the residue at an external temperature of 50°C and the addition of 960 mL of IPE. The seed crystals of the intended compound were added to the mixture, it was confirmed that the crystals were separated from the mixture, then the mixture was stirred at an external temperature of 50°C for 10 minutes, 1.92 L of IPE was added thereto, followed by the stirring of the mixture at an external temperature of 50°C for 30 minutes. The content of the flask was gradually cooled (after the flask was disconnected from the evaporator), stirred at an internal temperature of not higher than 15°C for 30 minutes (the internal temperature was changed from 15°C to 7°C), and the resulting crystals were recovered through filtration, followed by the washing thereof with 720 mL of IPE and the removal of the liquid to thus give 309 g of (-)-2-amino-5-[2-chloro-4-(3-trifluoromethylphenylthio)phenyl]-2-methyl-1-pentanol hydrochloride as a crude and wet crystalline product. The resulting crude and wet crystalline product (309 g) of (-)-2-amino-5-[2-chloro-4-(3-trifluoromethylphenylthio)phenyl]-2-methyl-1-pentanol hydrochloride was dried at 40°C for one hour and 45 minutes using an air-blower and further dried at 60°C overnight likewise using an air-blower to thus give 249 g of (-)-2-amino-5-[2-chloro-4-(3-trifluoromethylphenylthio)phenyl]-2-methyl-1-pentanol hydrochloride in the form of crude crystals.

To a 2 L volume four-necked flask, there were added 249 g of the crude crystals obtained above, 249 mL of ethyl acetate and 498 mL of IPE, and the crystals were dissolved while heating and stirring the mixture at an external temperature of 50°C (at an internal temperature of 46°C, it was confirmed that the crystals were completely dissolved). After cooling the resulting solution at an external temperature of 27°C (internal temperature: 30°C), the solution was filtered (filtration under reduced pressure; filter: KTRIYAMA funnel having a diameter of 15 cm; receiving flask: 5 L volume four-necked flask), followed by the washing of the funnel with a mixed liquid comprising 249 mL of ethyl acetate and 498 mL of IPE. The resulting filtrate was heated and stirred at an external temperature of 47°C and 1.99 L of IPE was dropwise added to the filtrate at an internal temperature ranging from 39 to 45°C. After confirming that the crystals were separated from the filtrate (after 13 minutes from the completion of the dropwise addition: the crystallization was initiated at an internal temperature of 46°C), the filtrate was stirred at an internal temperature ranging from 46 to 47°C for 10 minutes and then heated and stirred at an external temperature of 57°C (the heating was continued till the internal temperature reached 57°C). Then, the filtrate was gradually cooled and stirred at an internal temperature of not higher than 15°C for 15 minutes (the internal temperature was changed from 15°C to 7°C). The crystals thus formed were filtered off, they were washed with 748 mL of IPE and then the liquid was removed from the same to thus give 325 g of (-)-2-amino-5-[2-chloro-4-(3-trifluoromethylphenylthio)phenyl]-2-methyl-1-pentanol hydrochloride as a wet product. The resulting wet (-)-2-amino-5-[2-chloro-4-(3-trifluoromethylphenylthio)phenyl]-2-methyl-1-pentanol hydrochloride (325 g) was dried at 40°C for 2 hours using an air-blower and further dried at 60°C overnight likewise using an air-blower to thus give 247 g (561 mmol; yield: 94%) of (-)-2-amino-5-[2-chloro-4-(3-trifluoromethylphenylthio)phenyl]-2-methyl- 1-pentanol hydrochloride as white powdery crystals.

¹H-NMR(CDCl₃, 400) MHz)δ 1.37(3H, s), 1.63-1.77(2H, m), 1.86(2H, t, J = 8.2 Hz), 2.72(2H, t, J = 7.8 Hz), 3.67(2H, t, J = 12.9 Hz), 4.78(1H, br s), 7.17(1H, dd, J= 8.1,1.8 Hz), 7.21(1H, d, J = 8.1 Hz), 7.33(1H, d, J = 1.7 Hz), 7.36-7.43(2H, m), 7.45-7.47(1H, m), 7.53(1H, s), 8.02(3H, br s).
[a]_{D}²⁶ : -3.9°(c 1.0,CHCl₃, 100 mm)

Furthermore, the (-)-2-amino-5-[2-chloro-4-(3-trifluoromethylphenylthio) phenyl]-2-methyl-1-pentanol hydrochloride prepared according to the method of this invention was found to have an extremely high optical purity of not less than 99.5%e.e.

### Industrial Applicability

The method according to the present invention makes it possible to prepare each of the compounds represented by the general formula (1) having an immune-suppressive activity as a single optical isomer having a high optical purity, and the method of the present invention also makes it possible to prepare a large quantity of the desired compound at a low cost. Accordingly, the present invention would be industrially quite useful.

## Claims

1. A method for producing a compound represented by the following general formula (1): 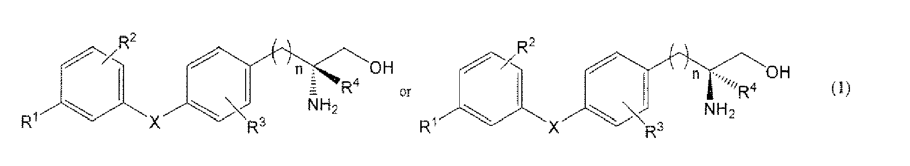 [wherein R¹ represents a halogen atom, a trihalomethyl group, a lower alkyl group having 1 to 4 carbon atoms, an aralkyl group, a lower alkoxy group having 1 to 4 carbon atoms, a phenoxy group which may have a substituent, an aralkyloxy group which may have a substituent, a lower alkylthio group having 1 to 4 carbon atoms, a lower alkylsulfinyl group having 1 to 4 carbon atoms, or a lower alkylsulfonyl group having 1 to 4 carbon atoms;
R² represents a hydrogen atom, a halogen atom, a trihalomethyl group, a lower alkyl group having 1 to 4 carbon atoms, an aralkyl group, a lower alkoxy group having 1 to 4 carbon atoms, or an aralkyloxy group;
R⁸ represents a hydrogen atom, a halogen atom, a trifluoromethyl group, or a lower alkylthio group having 1 to 4 carbon atoms;
R⁴ represents a hydrogen atom, a lower alkyl group having 1 to 4 carbon atoms, a mono-halogenomethyl group, a lower alkylthiomethyl group having 1 to 4 carbon atoms, a hydroxyethyl group, a hydroxypropyl group, a phenyl group, an aralkyl group, a lower alkenyl group having 2 to 4 carbon atoms, or a lower alkynyl group having 2 to 4 carbon atoms;
X represents an oxygen atom, a sulfur atom, SO, or SO₂; and
n represents an integer ranging from 1 to 4],
said method comprising the steps of:
(i) reacting a compound represented by the following general formula (2): 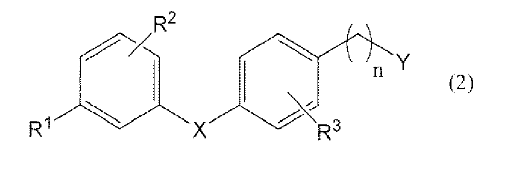 [wherein Y represents a halogen atom, an alkylsulfonyloxy group having 1 to 4 carbon atoms which may have a substituent, or an arylsulfonyloxy group which may have a substituent, and R¹, R², R³, X and n are the same as those defined above], with a compound represented by the following general formula (3): 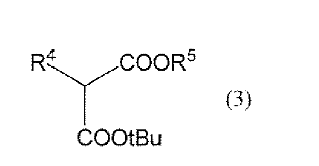 [wherein R⁵ represents a lower alkyl group having 1 to 4 carbon atoms which may have a substituent and R⁴ is the same as that defined above], to form a compound represented by the following general formula (4): 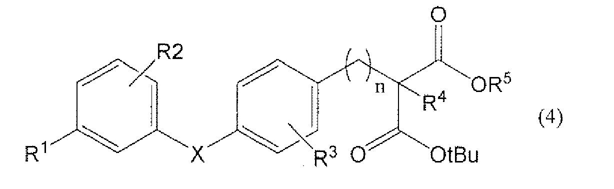 [wherein R¹, R², R³, R⁴ , R⁵, X and n are the same as those defined above];
(ii) replacing the t-butyl group of the compound represented by the general formula (4) with a hydrogen atom to form a compound represented by the following general formula (5): 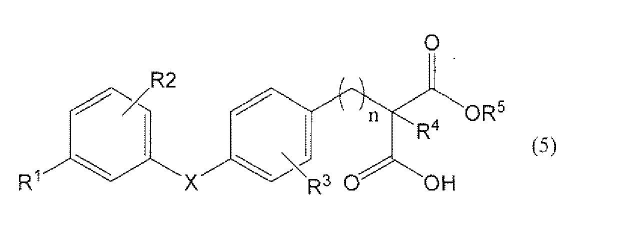 [wherein R¹, R², R³, R⁴, R⁵, X and n are the same as those defined above]>
(iii) subjecting the compound represented by the general formula (5) to a rearrangement reaction to give a compound represented by the following general formula (6): 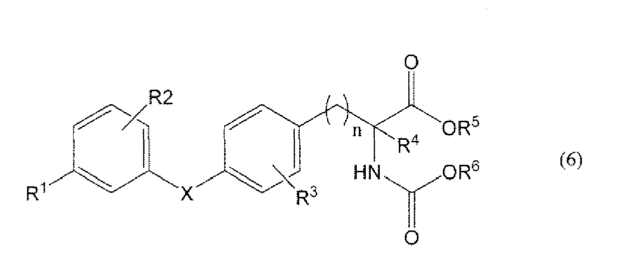 [wherein R⁶ represents a lower alkyl group having 1 to 4 carbon atoms which may have a substituent and R¹, R², R³, R⁴, R⁵, X and n are the same as those defined above];
(iv) optically resolving the compound represented by the general formula (6) to obtain a compound represented by the following general formula (7): 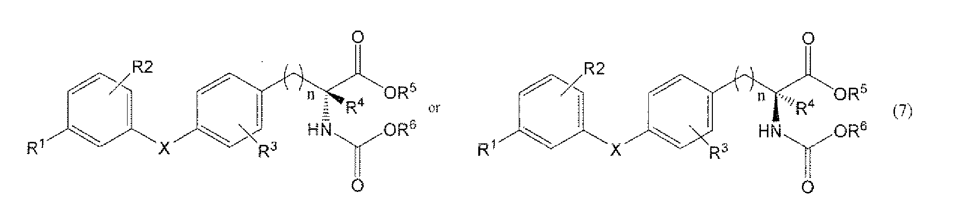 [wherein R¹, R² , R³, R⁴, R⁵, R⁶, X and n are the same as those defined above];
(v) hydrolyzing the compound represented by the general formula (7) to obtain a compound represented by the following general formula (8): 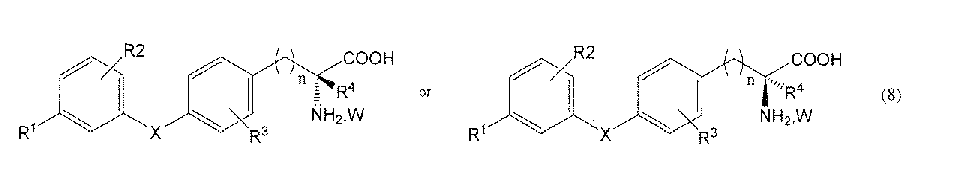 [wherein R¹, R², R³, R⁴, X and n are the same as those defined above and W represents hydrogen chloride or hydrogen bromide]; and
(vi) reducing the compound represented by the general formula (8) and then purifying the resulting reduced compound in the form of a salt thereof with fumaric acid or D- or L-tartaric acid to give a compound represented by the foregoing general formula (1).

2. The method as set forth in claim 1, wherein the optical resolution step (iv) makes use of an optical resolution column.

3. The method as set forth in claim 1 or 2, wherein the rearrangement reaction step (iii) is carried out using diphenyl phosphoryl azide.

4. The method as set forth in any one of claims 1 to 3, wherein X is a sulfur atom or an oxygen atom.

5. The method as set forth in claim 1, wherein W in the general formula (8) is hydrogen chloride.

6. The method as set forth in any one of claims 1 to 5, wherein the optical purity of the compound represented by the general formula (1) is not less than 99.5%e.e.

7. The method as set forth in any one of claims 1. to 6, wherein the salt in step (vi) is a salt with D- or L-tartaric acid.

8. A method for producing a compound represented by the following general formula (1-a): 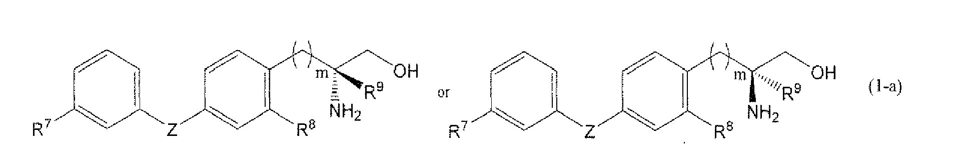 [wherein R⁷ represents a chlorine atom, a linear alkyl group having 1 to 3 carbon atoms, or a trifluoromethyl group;
R⁸ represents a fluorine atom or a chlorine atom;
R⁹ represents a linear alkyl group having 1 to 3 carbon atoms Z represents an oxygen atom or a sulfur atom;
m represents 2 or 3),
said method comprising the steps of:
(i) reacting a compound represented by the following general formula (2-a): 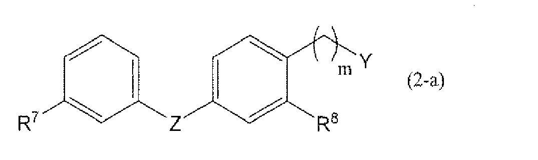 [wherein Y represents a halogen atom, an alkylsulfonyloxy group having 1 to 4 carbon atoms, which may have a substituent, or an arylsulfonyloxy group which may have a substituent, and R⁷, R⁸, Z and m are the same as those defined above], with a compound represented by the following general formula (3-a): 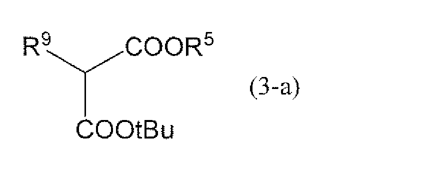 [wherein R⁵ represents a lower alkyl group having 1 to 4 carbon atoms which may have a substituent; and R⁹ is the same as that defined above], to obtain a compound represented by the following general formula (4.-a): 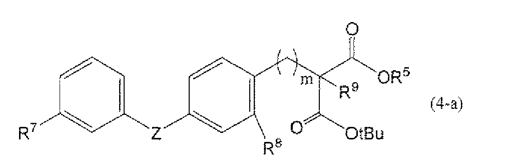 [wherein R⁵, R⁷, R⁸, R⁹, Z and m are the same as those defined above];
(ii) replacing the t-butyl group of the compound represented by the general formula (4-a) with a hydrogen atom to form a compound represented by the following general formula (5-a): 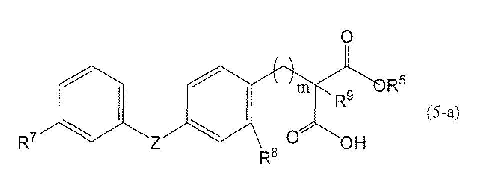 [wherein R⁵, R⁷, R⁸, R⁹, Z and m are the same as those defined above];
(iii) subjecting a compound represented by the general formula (5-a) to a rearrangement reaction to form a compound represented by the following general formula (6-a): 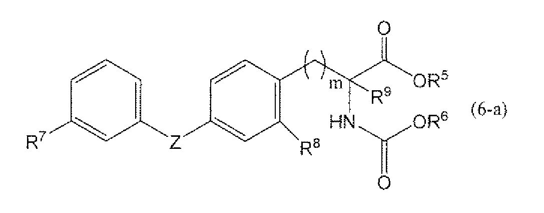 [wherein R⁶ represents a lower alkyl group having 1 to 4 carbon atoms which may have a substituent, and R⁵, R⁷, R⁸, R⁹, Z and m are the same as those defined above];
(iv) optically resolving the compound represented by the general formula (6-a), to form a compound represented by the following general formula (7-a): 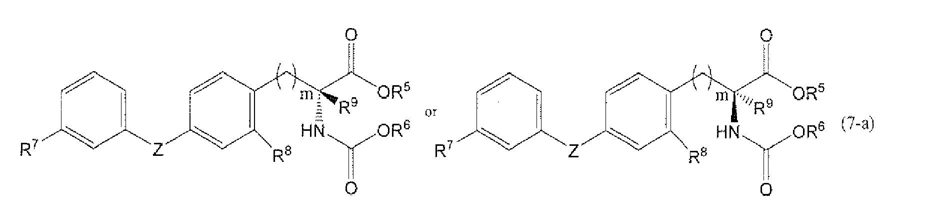 [wherein R⁵, R⁶, R⁷, R⁸, R⁹, Z and m are the same as those defined above];
(v) hydrolyzing the compound represented by the general formula (7-a) to form a compound represented by the following general formula (8-a) 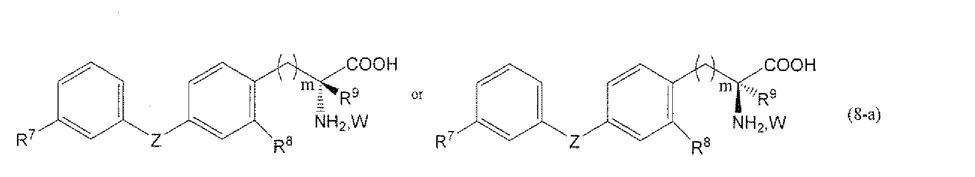 [wherein R⁷, R⁸, R⁹, Z and m are the same as those defined above, and W represents hydrogen chloride or hydrogen bromide]; and
(vi) reducing the compound represented by the general formula (8-a) and then purifying the resulting reduced compound in the form of a salt thereof with fumaric acid or D- or L-tartaric acid to give a compound represented by the foregoing general formula (1-a).

9. The method as set forth in claim 8, wherein the optical resolution step (iv) makes use of an optical resolution column.

10. The method as set forth in claim 8 or 9, wherein the rearrangement reaction step (iii) is carried out using diphenyl phosphoryl azide.

11. The method as set forth in any one of claims 8 to 10, wherein R⁷ is a trifluoromethyl group.

12. The method as set forth in any one of claims 8 to 11, wherein the optical purity of the compound represented by the general formula (1-a) is not less than 99.5%e.e.

13. The method as set forth in any one of claim 8 to 12, wherein the salt in step (vi) is a salt with D- or L-tartaric acid.

14. A method for improving the optical purity of a compound represented by the following general formula (8), comprising recrystallizing said compound: 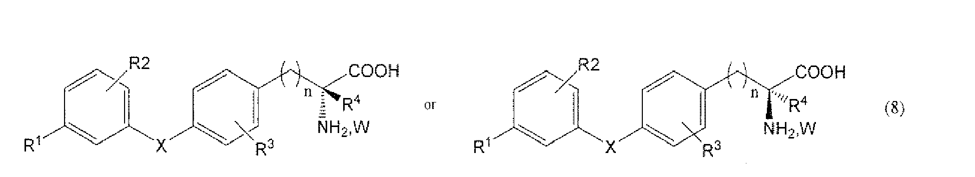 [wherein R¹ represents a halogen atom, a trihalomethyl group, a lower alkyl group having 1 to 4 carbon atoms, an aralkyl group, a lower alkoxy group having 1 to 4 carbon atoms, a phenoxy group which may have a substituent, an aralkyloxy group which may have a substituent, a lower alkylthio group having 1 to 4 carbon atoms, a lower alkylsulfinyl group having 1 to 4 carbon atoms, or a lower alkylsulfonyl group having 1 to 4 carbon atoms;
R² represents a hydrogen atom, a halogen atom, a trihalomethyl group, a lower alkyl group having 1 to 4 carbon atoms, an aralkyl group, a lower alkoxy group having 1 to 4 carbon atoms, or an aralkyloxy group;
R³ represents a hydrogen atom, a halogen atom, a trifluoromethyl group, or a lower alkylthio group having 1 to 4 carbon atoms;
R⁴ represents a hydrogen atom, a lower alkyl group having 1 to 4 carbon atoms, a mono-halogenomethyl group, a lower alkylthiomethyl group having 1 to 4 carbon atoms, a hydroxyethyl group, a hydroxypropyl group, a phenyl group, an aralkyl group, a lower alkenyl group having 2 to 4 carbon atoms, or a lower alkynyl group having 2 to 4 carbon atoms;
X represents an oxygen atom, a sulfur atom, SO, or SO₂;
n represents an integer ranging from 1 to 4; and
W represents hydrogen chloride or hydrogen bromide.

15. A method comprising the steps of:
(i) reducing a compound represented by the following general formula (8): 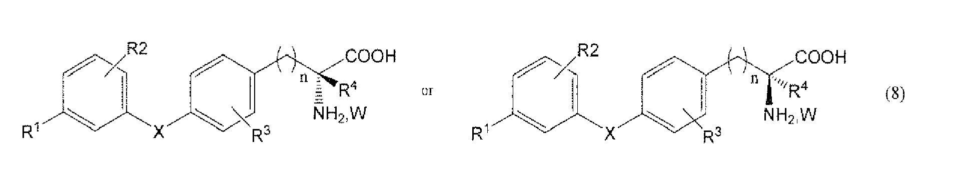 [wherein R¹ represents a halogen atom, a trihalomethyl group, a lower alkyl group having 1 to 4 carbon atoms, an aralkyl group, a lower alkoxy group having 1 to 4 carbon atoms, a phenoxy group which may have a substituent, an aralkyloxy group which may have a substituent, a lower alkylthio group having 1 to 4 carbon atoms, a lower alkylsulfinyl group having 1 to 4 carbon atoms, or a lower alkylsulfonyl group having 1 to 4 carbon atoms;
R² represents a hydrogen atom, a halogen atom, a trihalomethyl group, a lower alkyl group having 1 to 4 carbon atoms, an aralkyl group, a lower alkoxy group having 1 to 4 carbon atoms, or an aralkyloxy group;
R³ represents a hydrogen atom, a halogen atom, a trifluoromethyl group, or a lower alkylthio group having 1 to 4 carbon atoms;
R⁴ represents a hydrogen atom, a lower alkyl group having 1 to 4 carbon atoms, a mono-halogenomethyl group, a lower alkylthiomethyl group having 1 to 4 carbon atoms, a hydroxyethyl group, a hydroxypropyl group, a phenyl group, an aralkyl group, a lower alkenyl group having 2 to 4 carbon atoms, or a lower alkynyl group having 2 to 4 carbon atoms;
X represents an oxygen atom, a sulfur atom, SO, or SO₂;
n represents an integer ranging from 1 to 4; and
W represents hydrogen chloride or hydrogen bromide], and then
(ii) purifying the reduced compound by converting it to a salt with fumaric acid or D- or L-tartaric acid to obtain a compound represented by the following general formula (1): 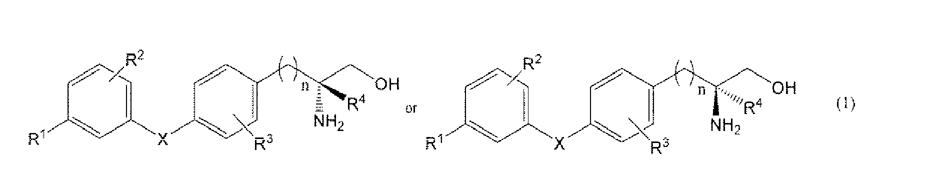 [wherein R¹, R², R³, R⁴, X and n are the same as those defined above].

16. The method as set forth in claim 15 wherein the salt in the purification step is a salt of the compound with D- or L-tartaric acid.

17. A compound represented by the following general formula (8-b): 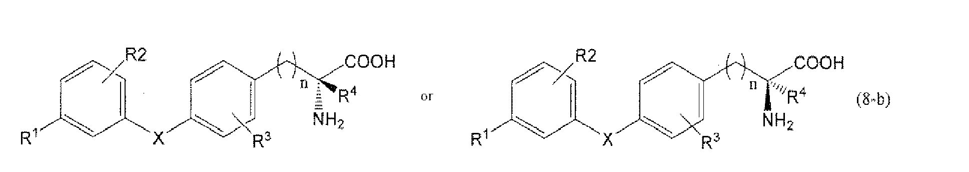 [wherein R¹ represents a halogen atom, a trihalomethyl group, a lower alkyl group having 1 to 4 carbon atoms, an aralkyl group, a lower alkoxy group having 1 to 4 carbon atoms, a phenoxy group which may have a substituent, an aralkyloxy group which may have a substituent, a lower alkylthio group having 1 to 4 carbon atoms, a lower alkylsulfinyl group having 1 to 4 carbon atoms, or a lower alkylsulfonyl group having 1 to 4 carbon atoms;
R² represents a hydrogen atom, a halogen atom, a trihalomethyl group, a lower alkyl group having 1 to 4 carbon atoms, an aralkyl group, a lower alkoxy group having 1 to 4 carbon atoms, or an aralkyloxy group;
R³ represents a hydrogen atom, a halogen atom, a trifluoromethyl group, or a lower alkylthio group having 1 to 4 carbon atoms;
R⁴ represents a hydrogen atom, a lower alkyl group having 1 to 4 carbon atoms, a mono-halogenomethyl group, a lower alkylthiomethyl group having 1 to 4 carbon atoms, a hydroxyethyl group, a hydroxypropyl group, a phenyl group, an aralkyl group, a lower alkenyl group having 2 to 4 carbon atoms, or a lower alkynyl group having 2 to 4 carbon atoms;
X represents an oxygen atom, a sulfur atom, SO, or SO₂; and
n represents an integer ranging from 1 to 4], or a salt thereof.
